# EUROPÄISCHE PATENTSCHRIFT

(11) **EP 0 885 962 B1**
(45) Veröffentlichungstag und Bekanntmachung des Hinweises auf die Patenterteilung: **13.04.2005**
(21) Anmeldenummer: 98109269.5
(22) Anmeldetag: 22.05.1998
(51) Int. Cl.: C12N 15/31, C12N 1/21, C12P 13/12, C07K 14/245

(54) **Mikroorganismen und Verfahren zur fermentativen Herstellung von L-Cystein, L-Cystin, N-Acetyl-Serin oder Thiazolindinderivaten**
Microorganisms and process for the fermentative production of L-Cystein, L-Cystin, N-Acetyl-Serin or thiazolidin-derivates
Microorganismes et procédé de production de L-cystéine, L-cystine, N-acetyl-sérine ou dérivés de thiazolidine par fermentation

(30) Priorität: 19.06.1997 DE 19726083
(43) Veröffentlichungstag der Anmeldung: 23.12.1998
(73) Patentinhaber: Consortium für elektrochemische Industrie GmbH, 81379 München (DE)
(72) Erfinder: Winterhalter, Christopher, Dr., 82343 Pöcking (DE); Leinfelder, Walfred, Dr., 81549 München (DE)
(74) Vertreter: Potten, Holger

(56) Entgegenhaltungen:
- DE-A- 19 539 952
- DE-A- 19 548 222
- NILSEN I ET AL: "Isolation of cmr, a novel Escherichia coli chloramphenicol resistance gene encoding a putative eflux pump" J. BACTERIOL., Bd. 178, Nr. 11, Juni 1996, Seiten 3188-3193, XP002078491
- DATABASE EMPRO E.M.B.L. Databases Accession Number: D90797, 21. Dezember 1996 MORI H: "E. coli genomic DNA" XP002078494
- VRLJIC M ET AL: "A NEW TYPE OF TRANSPORT WITH A NEW TYPE OF CELLULAR FUNCTION: L-LYSINE EXPORT FROM CORYNEBACTERIUM GLUTAMICUM" MOLECULAR MICROBIOLOGY, Bd. 22, Nr. 5, Dezember 1996, Seiten 815-826, XP000675494
- COHEN S ET AL: "Genetic and functional analysis of the multiple antibiotic resistance (mar) locus in Escherichia coli" J. BACTERIOL., Bd. 175, Nr. 5, März 1993, Seiten 1484-1492, XP002078492
- PAULSEN I ET AL: "Proton-dependent multidrug efflux systems" MICROBIOLOGICAL REVIEWS, Bd. 60, Dezember 1996, Seiten 575-608, XP002078493

### SEQUENZPROTOKOLL

(1) ALLGEMEINE ANGABEN:
   (i) ANMELDER:
      (A) NAME: Consortium fuer elektrochemische Industrie GmbH
      (B) STRASSE: Zielstattstr. 20
      (C) ORT: Muenchen
      (D) BUNDESLAND: Bayern
      (E) LAND: Deutschland
      (F) POSTLEITZAHL: 81379
      (G) TELEFON: 089-74844-0
      (H) TELEFAX: 089-74844-350
   (ii) BEZEICHNUNG DER ERFINDUNG: Mikroorganismen und Verfahren zur fermentativen Herstellung von L-Cystein, L-Cystin, N-Acetyl-Serin oder Thiazolidinderivaten
   (iii) ANZAHL DER SEQUENZEN: 11
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 2:
   (i) SEQUENZKENNZEICHEN:
      (A) LÄNGE: 306 Aminosäuren
      (B) ART: Aminosäure
      (C) STRANGFORM:
      (D) TOPOLOGIE: linear
   (ii) ART DES MOLEKÜLS: Protein
   (iii) HYPOTHETISCH: NEIN
   (iv) ANTISENSE: NEIN
   (vi) URSPRÜNLICHE HERKUNFT:
      (A) ORGANISMUS: Escherichia coli
      (B) STAMM: K12
   (xi) SEQUENZBESCHREIBUNG: SEQ ID NO: 2:
   Met Lys Phe Arg Gly Gly Arg Met Ser Arg Lys Asp Gly Val Leu Ala
   1 5 10 15
   Leu Leu Val Val Val Val Trp Gly Leu Asn Phe Val Val Ile Lys Val
   20 25 30
   Gly Leu His Asn Met Pro Arg Leu Met Leu Ala Gly Leu Arg Phe Met
   35 40 45
   Leu Val Ala Phe Pro Ala Ile Phe Phe Val Ala Arg Pro Lys Val Pro
   50 55 60
   Leu Asn Leu Leu Leu Gly Tyr Gly Leu Thr Ile Ser Phe Ala Gln Phe
   65 70 75 80
   Ala Phe Leu Phe Cys Ala Ile Asn Phe Gly Met Pro Ala Gly Leu Ala
   85 90 95
   Ser Leu Val Leu Gln Ala Gln Ala Phe Phe Thr Ile Met Leu Gly Ala
   100 105 110
   Phe Thr Phe Gly Glu Arg Leu His Gly Lys Gln Leu Ala Gly Ile Ala
   115 120 125
   Leu Ala Ile Phe Gly Val Leu Val Leu Ile Glu Asp Ser Leu Asn Gly
   130 135 140
   Gln His Val Ala Met Leu Gly Phe Met Leu Thr Leu Ala Ala Ala Phe
   145 150 155 160
   Ser Trp Ala Cys Gly Asn Ile Phe Asn Lys Lys Ile Met Ser His Ser
   165 170 175
   Thr Arg Pro Ala Val Met Ser Leu Val Ile Trp Ser Ala Leu Ile Pro
   180 185 190
   Ile Ile Pro Phe Phe Val Ala Ser Leu Ile Leu Asp Gly Ser Ala Thr
   195 200 205
   Met Ile His Ser Leu Val Thr Ile Asp Met Thr Thr Ile Leu Ser Leu
   210 215 220
   Met Tyr Leu Ala Phe Val Ala Thr Ile Val Gly Tyr Gly Ile Trp Gly
   225 230 235 240
   Thr Leu Leu Gly Arg Tyr Glu Thr Trp Arg Val Ala Pro Leu Ser Leu
   245 250 255
   Leu Val Pro Val Val Gly Leu Ala Ser Ala Ala Leu Leu Leu Asp Glu
   260 265 270
   Arg Leu Thr Gly Leu Gln Phe Leu Gly Ala Val Leu Ile Met Thr Gly
   275 280 285
   Leu Tyr Ile Asn Val Phe Gly Leu Arg Trp Arg Lys Ala Val Lys Val
   290 295 300
   Gly Ser
   305
(2) ANGABEN ZU SEQ ID NO: 3:
   (i) SEQUENZKENNZEICHEN:
      (A) LÄNGE: 299 Aminosäuren
      (B) ART: Aminosäure
      (C) STRANGFORM:
      (D) TOPOLOGIE: linear
   (ii) ART DES MOLEKÜLS: Protein
   (iii) HYPOTHETISCH: NEIN
   (iv) ANTISENSE: NEIN
   (vi) URSPRÜNLICHE HERKUNFT:
      (A) ORGANISMUS: Escherichia coli
      (B) STAMM: K12
   (xi) SEQUENZBESCHREIBUNG: SEQ ID NO: 3:
   Met Ser Arg Lys Asp Gly Val Leu Ala Leu Leu Val Val Val Val Trp
   1 5 10 15
   Gly Leu Asn Phe Val Val Ile Lys Val Gly Leu His Asn Met Pro Arg
   20 25 30
   Leu Met Leu Ala Gly Leu Arg Phe Met Leu Val Ala Phe Pro Ala Ile
   35 40 45
   Phe Phe Val Ala Arg Pro Lys Val Pro Leu Asn Leu Leu Leu Gly Tyr
   50 55 60
   Gly Leu Thr Ile Ser Phe Ala Gln Phe Ala Phe Leu Phe Cys Ala Ile
   65 70 75 80
   Asn Phe Gly Met Pro Ala Gly Leu Ala Ser Leu Val Leu Gln Ala Gln
   85 90 95
   Ala Phe Phe Thr Ile Met Leu Gly Ala Phe Thr Phe Gly Glu Arg Leu
   100 105 110
   His Gly Lys Gln Leu Ala Gly Ile Ala Leu Ala Ile Phe Gly Val Leu
   115 120 125
   Val Leu Ile Glu Asp Ser Leu Asn Gly Gln His Val Ala Met Leu Gly
   130 135 140
   Phe Met Leu Thr Leu Ala Ala Ala Phe Ser Trp Ala Cys Gly Asn Ile
   145 150 155 160
   Phe Asn Lys Lys Ile Met Ser His Ser Thr Arg Pro Ala Val Met Ser
   165 170 175
   Leu Val Ile Trp Ser Ala Leu Ile Pro Ile Ile Pro Phe Phe Val Ala
   180 185 190
   Ser Leu Ile Leu Asp Gly Ser Ala Thr Met Ile His Ser Leu Val Thr
           195                 200                 205
   Ile Asp Met Thr Thr Ile Leu Ser Leu Met Tyr Leu Ala Phe Val Ala
       210                 215                 220
   Thr Ile Val Gly Tyr Gly Ile Trp Gly Thr Leu Leu Gly Arg Tyr Glu
   225                 230                 235                 240
   Thr Trp Arg Val Ala Pro Leu Ser Leu Leu Val Pro Val Val Gly Leu
                   245                 250                 255
   Ala Ser Ala Ala Leu Leu Leu Asp Glu Arg Leu Thr Gly Leu Gln Phe
               260                 265                 270
   Leu Gly Ala Val Leu Ile Met Thr Gly Leu Tyr Ile Asn Val Phe Gly
           275                 280                 285
   Leu Arg Trp Arg Lys Ala Val Lys Val Gly Ser
       290                 295
(2) ANGABEN ZU SEQ ID NO: 4:
   (i) SEQUENZKENNZEICHEN:
      (A) LÄNGE: 266 Aminosäuren
      (B) ART: Aminosäure
      (C) STRANGFORM:
      (D) TOPOLOGIE: linear
   (ii) ART DES MOLEKÜLS: Protein
   (iii) HYPOTHETISCH: NEIN
   (iv) ANTISENSE: NEIN
   (vi) URSPRÜNLICHE HERKUNFT:
      (A) ORGANISMUS: Escherichia coli
      (B) STAMM: K12
   (xi) SEQUENZBESCHREIBUNG: SEQ ID NO: 4:
   Met Leu Ala Gly Leu Arg Phe Met Leu Val Ala Phe Pro Ala Ile Phe
   1 5 10 15
   Phe Val Ala Arg Pro Lys Val Pro Leu Asn Leu Leu Leu Gly Tyr Gly
   20 25 30
   Leu Thr Ile Ser Phe Ala Gln Phe Ala Phe Leu Phe Cys Ala Ile Asn
   35 40 45
   Phe Gly Met Pro Ala Gly Leu Ala Ser Leu Val Leu Gln Ala Gln Ala
   50 55 60
   Phe Phe Thr Ile Met Leu Gly Ala Phe Thr Phe Gly Glu Arg Leu His
   65 70 75 80
   Gly Lys Gln Leu Ala Gly Ile Ala Leu Ala Ile Phe Gly Val Leu Val
   85 90 95
   Leu Ile Glu Asp Ser Leu Asn Gly Gln His Val Ala Met Leu Gly Phe
   100 105 110
   Met Leu Thr Leu Ala Ala Ala Phe Ser Trp Ala Cys Gly Asn Ile Phe
   115 120 125
   Asn Lys Lys Ile Met Ser His Ser Thr Arg Pro Ala Val Met Ser Leu
   130 135 140
   Val Ile Trp Ser Ala Leu Ile Pro Ile Ile Pro Phe Phe Val Ala Ser
   145 150 155 160
   Leu Ile Leu Asp Gly Ser Ala Thr Met Ile His Ser Leu Val Thr Ile
   165 170 175
   Asp Met Thr Thr Ile Leu Ser Leu Met Tyr Leu Ala Phe Val Ala Thr
   180 185 190
   Ile Val Gly Tyr Gly Ile Trp Gly Thr Leu Leu Gly Arg Tyr Glu Thr
   195 200 205
   Trp Arg Val Ala Pro Leu Ser Leu Leu Val Pro Val Val Gly Leu Ala
   210 215 220
   Ser Ala Ala Leu Leu Leu Asp Glu Arg Leu Thr Gly Leu Gln Phe Leu
   225 230 235 240
   Gly Ala Val Leu Ile Met Thr Gly Leu Tyr Ile Asn Val Phe Gly Leu
   245 250 255
   Arg Trp Arg Lys Ala Val Lys Val Gly Ser
   260 265
(2) ANGABEN ZU SEQ ID NO: 5:
   (i) SEQUENZKENNZEICHEN:
      (A) LÄNGE: 38 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "synthetic"
   (iii) HYPOTHETISCH: NEIN
   (iv) ANTISENSE: NEIN
   (xi) SEQUENZBESCHREIBUNG: SEQ ID NO: 5:
   TGGACCAGAG CTCTGGCTGG CGCATCGCTT CGGCGTTG 38
(2) ANGABEN ZU SEQ ID NO: 6:
   (i) SEQUENZKENNZEICHEN:
      (A) LÄNGE: 38 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "synthetic"
   (iii) HYPOTHETISCH: NEIN
   (iv) ANTISENSE: NEIN
   (xi) SEQUENZBESCHREIBUNG: SEQ ID NO: 6:
   CTCGATGCAT TACGTAGGGG TATCCGGGAG CGGTATTG 38
(2) ANGABEN ZU SEQ ID NO: 7:
   (i) SEQUENZKENNZEICHEN:
      (A) LÄNGE: 35 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "synthetic"
   (iii) HYPOTHETISCH: NEIN
   (iv) ANTISENSE: NEIN
   (xi) SEQUENZBESCHREIBUNG: SEQ ID NO: 7:
   TTTGGCGCGC CGATCAGCGG CGGCGCAACC ATCAG 35
(2) ANGABEN ZU SEQ ID NO: 8:
   (i) SEQUENZKENNZEICHEN:
      (A) LÄNGE: 38 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "synthetic"
   (iii) FiYFOTHETISCH: NEIN
   (iv) ANTISENSE: NEIN
   (xi) SEQUENZBESCHREIBUNG: SEQ ID NO: 8:
   GCCTTAATTA AGATCGACAC TCAGGCTGTA CTGGCGAC 38
(2) ANGABEN ZU SEQ ID NO: 9:
   (i) SEQUENZKENNZEICHEN:
      (A) LÄNGE: 35 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "synthetic"
   (iii) HYPOTHETISCH: NEIN
   (iv) ANTISENSE: NEIN
   (xi) SEQUENZBESCHREIBUNG: SEQ ID NO: 9:
   GGAATTCATT AATCCGGCGA CTAACGAATC AACTG 35
(2) ANGABEN ZU SEQ ID NO: 10:
   (i) SEQUENZKENNZEICHEN:
      (A) LÄNGE: 36 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "synthetic"
   (iii) HYPOTHETISCHE: NEIN
   (iv) ANTISENSE: NEIN
   (xi) SEQUENZBESCFiREIBUNG: SEQ ID NO: 10:
(2) ANGABEN ZU SEQ ID NO: 11:
   (i) SEQUENZKENNZEICHEN:
      (A) LÄNGE: 33 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "synthetic"
   (iii) HYPOTHETISCH: NEIN
   (iv) ANTISENSE: NEIN
   (xi) SEQUENZBESCHREIBUNG: SEQ ID NO: 11:
   GTCGACGCGT GAGGCGAGTC AGTCGCGTAA TGC 33
(2) ANGABEN ZU SEQ ID NO: 12:
   (i) SEQUENZKENNZEICHEN:
      (A) LÄNGE: 43 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "synthetic"
   (iii) HYPOTHETISCH: NEIN
   (iv) ANTISENSE: NEIN
   (xi) SEQUENZBESCHREIBUNG: SEQ ID NO: 12:
   GACCTTAATT AAGATCTCAT ATGTTCCACC AGCTATTTGT TAG 43

## Beschreibung

Die Erfindung betrifft Mikroorganismen und Verfahren zur fermentativen Herstellung von L-Cystein, L-Cystin, N-Acetyl-Serin oder Thiazolidinderivaten.

Die fermentative Herstellung von Aminosäuren ist inzwischen für viele Aminosäuren Stand der Technik. Es existiert bisher jedoch kein wirtschaftliches fermentatives Verfahren für die Herstellung von L-Cystein.

Thiazolidinderivate und die entsprechenden Hemithioketale entstehen allgemein bei der Kondensation von Cystein mit Ketonen oder Aldehyden. Die chemische Kondensation von Cystein mit verschiedenen Ketonen oder Aldehyden, insbesondere mit α-Ketosäuren, ist schon lange bekannt. Die Kondensation erfolgt über das Hemithioketal als Zwischenstufe, das durch den nukleophilen Angriff des freien Elektronenpaars des Schwefels am positivierten Kohlenstoffatom der Aldehyd- oder Ketogruppe entsteht. Ein Ringschluß unter Wasserabspaltung führt dann zum entsprechenden Thiazolidinderivat.

Im folgenden Schema ist die Bildung von Thiazolidinderivaten allgemein dargestellt: 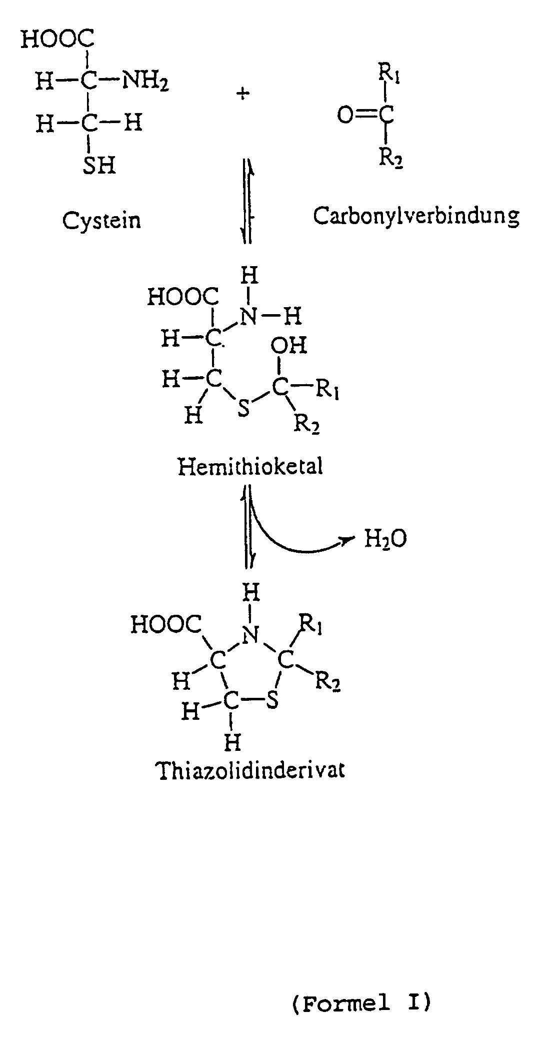 R₁ und R₂ können dabei beliebige organische Reste bedeuten.

Die Edukte stehen somit über das Hemithioketal mit dem Thiazolidinderivat im Gleichgewicht. Daher kommt in wäßriger Lösung neben dem Thiazolidinderivat im allgemeinen auch das Hemithioketal vor.

Im Sinne der vorliegenden Erfindung ist mit "Thiazolidinderivat" auch ein Gleichgewicht dieser Substanzen mit dem dazugehörigen Hemithioketal gemeint.

Thiazolidine wurden bisher nicht als direkte Metaboliten von Zellen beschrieben. Alle Berichte von Thiazolidinbildungen durch Zellen beruhen auf der übermäßigen externen Zugabe von einem der Edukte, meistens von L-Cystein, das dann durch Desulfhydrierung und Desaminierung in Pyruvat umgewandelt wird, welches wiederum mit dem zugegebenen Cystein reagiert. (Ronald C. Simpson et al., Biochimica et Biophysic Acta, 496 (1977), 12-19). Kredich et al. beschrieben in J. of Biol. Chem. 248, 17: 6187-6196, die in vitro Bildung von 2-Methyl-2,4-Thiazolidindicarbonsäure bei einer enzymatischen Desulfhydrierung von L-Cystein, halten jedoch die Bildung dieser Substanz in vivo für äußerst unwahrscheinlich.

Es ist bekannt, Thiazolidine als racemische Vorstufen für die Herstellung von L-Cystein durch Biotransformation zu verwenden (EP-A 0 101 052, Ok Hee Ryu et al., Biotechnology Letters 17 Nr. 3, 275-280 (März 1995)). Wenn das Racemat zur Herstellung von L-Cystein eingesetzt wird, muß dies durch Enzyme oder ganze Zellen stereoselektiv zu L-Cystein umgewandelt werden. Die verbleibenden Diastereomere müssen anschließend wieder racemisiert werden. Diese Biotransformation ist aus diesen Gründen mit hohen Kosten belastet.

Die chemische Synthese von Thiazolidinen aus racemischem Cystein und einem entsprechenden Keton oder Aldehyd führt zu vier verschiedenen Diastereomeren. Eine chemische Synthese aus enantiomerenreinem L-Cystein ist teuer und zum Zweck der anschließenden Gewinnung von L-Cystein unsinnig. Daher leidet ein Verfahren zur Herstellung von Thiazolidindiastereomeren, die am C4-Atom die R-Konfiguration aufweisen unter den hohen Kosten der Edukte.

Die vorliegende Erfindung betrifft einen Mikroorganismen stamm der zur fermentativen Herstellung von L-Cystein, L-Cystin, N-Acetyl-Serin und/oder Thiazolidinderivaten geeignet ist, dadurch gekennzeichnet, daß er in seinem Cysteinstoffwechsel so dereguliert ist, daß er eine erhöhte Menge an L-Cystein bildet und mindestens ein Gen kodierend für ein Protein mit SEQ.ID.NO: 2 oder eine Variante davon, welche zur Herstellung von L-Cystein, L-Cystin, N-Acetyl-Serin und/oder Thiazolidinderivaten beiträgt.

Die Erfindung betrifft ferner Proteine umfassend die Sequenz oder eine Variante der Sequenz welche [zur Herstellung von L-Cystein, L-Cystin, N-Acetyl-Serin und/oder Thiazolidinderivaten] beiträgt. Der offene Leserahmen, der für das Protein mit der Aminosäuresequenz gemäß SEQ. ID. NO: 2 kodiert, wird im folgenden auch als ORF 306 bezeichnet.

Ein weiteres Beispiel für ein erfindungsgemäßes Protein gibt die folgende Sequenz wieder.

Erfindungsgemäße Proteine sind auch solche Proteine die eine Aminosäuresequenz mit einer Sequenzhomologie auf Aminosäureebene größer als 50 % zur Aminosäuresequenz gemäß SEQ. ID. NO: 2 oder SEQ. ID. NO: 3 besitzen soweit sie zur Herstellung von L-Cystein, L-Cystin, N-Acetyl-Serin und/oder Thiazolidinderivaten beitragen

Vorzugsweise ist die Sequenzhomologie der erfindungsgemäßen Proteine zu SEQ. ID. NO: 2 oder SEQ. ID. NO: 3 größer 75 %, besonders bevorzugt ist die Sequenzhomologie zu SEQ. ID. NO: 2 oder SEQ. ID. NO : 3 größer 90% soweit sie zur Herstellung von L-Cystein, L-Cystin, N-Acetyl-Serin und/oder Thiazolidinderivaten beitragen

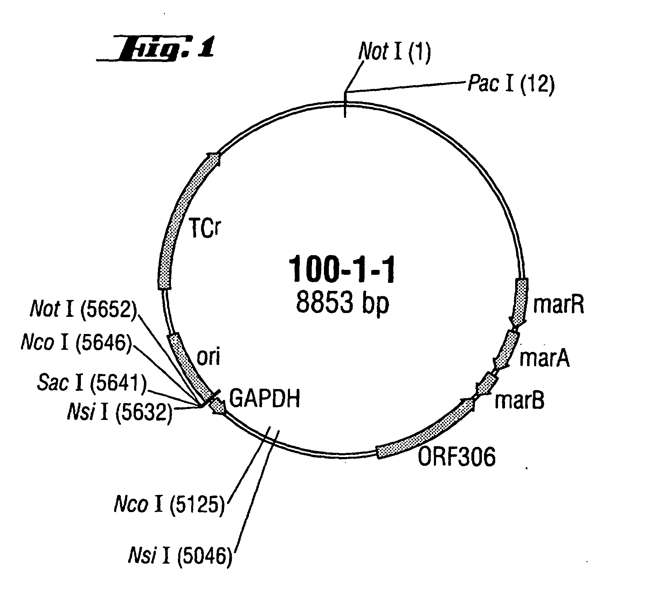

Ein weiteres Beispiel für die Überexpression eines Gens zur Steigerung der Cysteinbildung ist die Überexpression eines 5,5 kb langen DNS-Fragments, das auch für den mar-Locus kodiert. Dieses Plasmid mit der Bezeichnung 100-1-1 wurde bei der DSMZ Deutschen Sammlung von Mikroorganismen und Zellkulturen GmbH, D-38124 Braunschweig in E.coli K12 W3110 unter der Nummer DSM 11545 hinterlegt. Fig. 1 zeigt eine Plasmidkarte dieses Plasmids. Dieses Plasmid kann zur Amplifikation von erfindungsgemäßen Gene mittels PCR genutzt werden.

Eine gezielte weitere Modifikation dieser Gene an jeweils gewünschter Position der Sequenz mittels bekannter Verfahren, beispielsweise der Technik der site directed Mutagenese ist dem Fachmann geläufig. Auch Mikroorganismen, die derart modifizierte Gene enhalten sind demnach erfindungsgemäß solange die derart modifizierten Gene zur Herstellung von L-Cystein, L-Cystin, N-Acetyl-Serin und/oder Thiazolidinderivaten beitragen.

Unter Überexpression ist im Sinne der Erfindung zu verstehen, daß die Expression des Proteins im erfindungsgemäßen Mikroorganismus mindestens doppelt so stark erfolgt wie im Wildtyp aus dem das Protein stammt.

Vorzugsweise erfolgt die Expression des Proteins im erfindungsgemäßen Mikroorganismus mindestens fünfmal so stark wie im Wildtyp, besonders bevorzugt mindestens zehnmal so stark wie im Wildtyp aus dem das Protein stammt.

Ohne eine Überexpression der genannten Gene beispielsweise durch unabhängige Transkription durch einen gesonderten Promotor oder beispielsweise ohne Anwesenheit des für MarA kodierenden Gens in vielen Kopien auf einem Plasmid ist keine deutliche Ausbeutesteigerung für L-Cystein oder Thiazolidinderivate gegenüber dem Ausgangsstamm zu beobachten.

Die Ausbeutesteigerung durch die Überexpression der genannten Sequenzen war um so überraschender, als das in der Literatur beschriebene Genprodukt des offenen Leserahmens ORF266 dessen Sequenz ab dem Methionin in Position 41 in SEQ: ID: NO: 2 der Sequenz SEQ: ID: NO: 4 entspricht, bei Überexpression nicht zu einer Ausbeutesteigerung an L-Cystein führt.

In der oben dargestellten von ORF306 abgeleiteten Aminosäuresequenz ist das Startmethionin von ORF266 fett gedruckt und unterstrichen.

Dem Fachmann sind eine Reihe von Verfahren bekannt, die Überexpression eines Gens zu erreichen. Eine Möglichkeit ist beispielsweise die Expression des Gens auf einem Plasmid, das mit einer erhöhten Kopiezahl in der Zelle vorliegt. Solche Plasmide sind bekannt. Beispielsweise seien genannt pACYC177, pACYC184, Derivate von pACYC184, pBR322, andere pBR-Derivate, pBlueskript, pUC18, pUC19 sowie andere in Escherichia coli herkömmlich verwendete Plasmide.

Bevorzugte Plasmide zur erfindungsgemäßen Überexpression sind pACYC177, pACYC184, Derivate von pACYC184, pBR322 und andere pBR-Derivate.

Besonders bevorzugt werden pACYC184 und dessen Derivate wie beispielsweise pACYC184-LH (hinterlegt bei der DSMZ Deutschen Sammlung von Mikroorganismen und Zellkulturen GmbH, D-38124 Braunschweig unter der Nummer DSM 10172).

Weitere Möglichkeiten für eine Expressionsverstärkung sind die Erhöhung der Kopienzahl eines der genannten Gene durch eine Amplifizierung des Genabschnitts im Chromosom oder die Verwendung von starken Promotoren zur besseren Transkription des Efflux-Gens.

Als starke Promotoren sind beispielsweise der GAPDH-Promotor, der tac Promotor (p_{tac}), der Lac-Promotor (p_{lac}), der trp Promotor (pₜᵣₚ), Lambda PL oder Lambda PR geeignet.

Bevorzugt geeignet sind der GAPDH-Promotor oder der tac Promotor (p_{tac}).

Besonders bevorzugt geeignet ist der GAPDH-Promotor.

Eine weitere Möglichkeit für eine Expressionsverstärkung ist die Inaktivierung von Repressorgenen, die auf die Expression eines der genannten Gene hemmend wirken. Für den mar-Genort wäre dies beispielsweise die Inaktivierung des mar R-Gens.

Auch Elemente, die die Translation positiv beeinflussen, tragen zu einer Überexpression der genannten Gene bei. Solche Elemente sind beispielsweise eine gute Ribosomenbindungsstelle (z. B. Shine-Dalgarno Sequenz) oder Downstream Box.

Ein bevorzugtes Element, das die Translation positiv beeinflußt ist die gute Ribosomenbindungsstelle des GAPDH-Gens.

Zur Expression der genannten Gene werden diese in einen Mikroorganismus transformiert, der L-Cystein produziert.

Vorzugsweise werden die Gene in Mikrorganismen transformiert ausgewählt aus der Gruppe Bacillus wie B.subtilis, Corynebacterium wie C. glutamicum, Streptomyces und E. coli.

Die Gene werden in Organismen transformiert, die in ihrem Cysteinstoffwechsel so dereguliert sind, daß es zur Bildung von erhöhten Mengen an L-Cystein und ggf. nachfolgend zur Bildung eines Thiazolidinderivats von L-Cystin oder von N-Acetyl-Serin kommt.

Beispiele für Mikroorganismen die erhöhte Mengen an L-Cystein produzieren sind Mikroorganismen mit feedbackresistentem CysE Allel.

In einer weiteren bevorzugten Ausführungsform werden Mikroorganismen transformiert, die intrazellulär durch die Kondensation von L-Cystein und einem Keton oder Aldehyd, insbesondere Pyruvat, ein Thiazolidinderivat bilden.

Mikroorganismen, die erhöhte Mengen an L-Cystein produzieren sind beispielsweise in der Patententanmeldung DE 19539952 beschrieben.

Verfahren zur Transformation eines Mikroorganismus sind dem Fachmann beispielsweise aus Standardlehrbüchern bekannt. Alle bekannten Verfahren lassen sich zur Herstellung eines erfindungsgemäßen Mikroorganismus anwenden.

Durch eine verstärkte Expression der genannten Gene in Mikroorganismen, die Aminosäuren oder intrazellulär gebildete Aminosäurederivate wie beispielsweise L-Cystein, L-Cystin, N-Acetyl-Serin oder Thiazolidinderivate hiervon produzieren, kommt es überraschenderweise zu einer verstärkten Ausschleusung von Aminosäuren oder intrazellulär gebildeten Aminosäurederivaten wie z.B. L-Cystein, L-Cystin, N-Acetyl-Serin und Thiazolidinderivaten hiervon aus der Zelle. Dadurch werden deutlich höhere Ausbeuten dieser Produkte in der Fermentation erzielt.

Die Erfindung betrifft somit auch Verfahren zur Herstellung von L-Cystein, L-Cystin, N-Acetyl-Serin oder Thiazolidinderivaten hiervon dadurch gekennzeichnet, daß ein Mikroorganismus gemäß Anspruch 1 oder 2 in an sich bekannter Art und Weise in der Fermentation eingesetzt wird.

Das erfindungsgemäße Verfahren zur fermentativen Herstellung von L-Cystein, L-Cystin, N-Acetyl-Serin oder Thiazolidinderivaten weist mehrere Vorteile auf:

Es entstehen nur Thiazolidindiastereomere, die am C4-Kohlenstoffatom nur die R-Konfiguration aufweisen, da durch die Enzymausstattung der Zelle stereoselektiv L-Cystein entsteht, das dann mit dem jeweiligen verfügbaren Keton oder Aldehyd ausschließlich zu den genannten Thiazolidindiastereomeren reagieren kann.

Aus den Thiazolidinen mit der R-Konfiguration am C4-Kohlenstoffatom kann unter Verwendung von herkömmlichen chemischen und biologischen Verfahren und Techniken lediglich durch Gleichgewichtsverschiebung in Richtung der Edukte L-Cystein gewonnen werden.

Vorzugsweise bedeutet für die im erfindungsgemäßen Verfahren entstehenden Thiazolidinderivate im Schema auf S. 2 mindestens ein Rest R₁ oder R₂ Carboxylgruppe, besonders bevorzugt steht in Formel I R₁ für COOH und R₂ für CH₃.

Die Edukte für die Kondensation zum Thiazolidinderivat können entweder beide von dem Mikroorganismus gebildet werden oder es wird nur ein Edukt von dem Mikroorganismus gebildet und das zweite Edukt wird während der Fermentation zugegeben.

In einer bevorzugten Ausführungsform der Erfindung werden beide Edukte für die Kondensation zum Thiazolidinderivat durch den Mikroorganismus gebildet.

Als Derivatisierungsmittel zur Derivatisierung von Pyruvat, das so dem Gleichgewicht entzogen werden kann, können unter anderem Hydroxylamin oder 2,4-Dinitrophenylhydrazin verwendet werden.

Vorteilhafterweise können im erfindungsgemäßen Verfahren das Thiazolidinderivat (und das entsprechende Hemithioketal) aus einfachen und billigen C- und N- und S-Quellen hergestellt werden.

Es können die, in der Fermantation üblichen C-Quellen, wie beispielsweise Glucose, Lactose, Fructose, Stärke und dergleichen, N-Quellen, wie beispielsweise Ammonium oder Proteinhydrolysate und dergleichen, und S-Quellen, wie beispielsweise Sulfid, Sulfit, Sulfat, Thiosulfat oder Dithionit im erfindungsgemäßen Verfahren in der Fermentation verwendet werden.

Die fermentativ gewonnenen Thiazolidinderivate können nicht nur zur Gewinnung von Cystein eingesetzt werden. Es sind viele Einsatzmöglichkeiten bekannt, die die fermentativ hergestellten Thiazolidinderivate mit der R-Konfiguration am C4-Kohlenstoffatom als Ausgangsprodukt für weitergehende Synthesen (building block) verwenden können.

Die folgenden Beispiele dienen zur weiteren Erläuterung der Erfindung.

Die quantitative Bestimmung an Thiazolidinderivat/Hemithioketal ist nur indirekt möglich. Sie erfolgte in den Beispielen durch die Cysteinbestimmung nach Gaitonde, M. K. (1967), Biochem. J. 104, 627 - 633. Durch die Derivatisierung des Cysteins im stark Sauren mit Ninhydrin wird dies dem Gleichgewicht entzogen. Somit reagiert das Hemithioketal und schließlich auch das Thiazolidinderivat nach. Nach etwa 10 Minuten bei 100 °C wird das gesamte Thiazolidinderivat und das dazugehörige Hemithioketal in das Cystein-Ninhydrinderivat überführt, das dann bei 560 nm quantifiziert werden kann. Freies Cystein wird hierbei miterfaßt.

Die Menge an freien SH-Gruppen und somit freiem Cystein allein wurde durch den von Sang-Han Lee et al., Biochemical and Biophysical Research Communications, Vol. 213, Nr. 3 (1995), Seiten 837ff beschriebenen Test mittels 5,5'-Dithiobis-2-nitrobenzoesäure (DTNB) bestimmt.

Bei einer Bildung von freiem L-Cystein wird dies durch den eingebrachten Luftsauerstoff während der Fermentation zum L-Cystin oxidiert. Cystin ist im wäßrigen Milieu bei pH 7,0 schwer löslich und fällt als weißes Pellet aus. Bei Ausbildung eines unlöslichen Cystinpellets wurde dies in halbkonzentrierter HCl aufgelöst und ebenfalls unter reduzierenden Bedingungen mit Dithiothreit (DTT) im oben erwähnten Test vermessen.

Im Beispiel 3 sind als Fermentationsergebnisse die im Überstand mit dem Test nach Gaitonde gemessenen Mengen an "Gesamtcystein" angegeben. Dies sind hierbei vor allem 2-Methylthiazolidin-2,4-dicarbonsäure, das zugehörige Hemithioketal, freies L-Cystein und gelöstes Cystin. Ausgefallenes Cystin wurde gesondert quantifiziert und angegeben.

Die leichte Fällbarkeit, der in der Ausführungsform der vorliegenden Erfindung entstehenden 2-Methyl-thiazolidin-2,4-dicarbonsäure mittels zweiwertiger Metallionen kann beim Nachweis für die Entstehung dieses Derivats ausgenutzt werden. Bisher wurde nur die Fällbarkeit mit Zinkacetat beschrieben (Schubert et. al, siehe obige Literaturstelle). Eine Fällung mit anderen zweiwertigen Metallionen, wie Magnesium, Eisen, Kupfer, Zink, Mangan, Cobalt und dergleichen ist aber ebenfalls möglich. Die Fällung und anschließende Identifizierung des gebildeten Thiazolidinprodukts ist in Beispiel 4 beschrieben. Hier wird auch gezeigt, daß nach 24 Stunden Fermentationszeit das Hauptprodukt 2-Methyl-thiazolidin-2,4-dicarbonsäure ist. Die leichte Fällbarkeit ist nicht nur bei der Analyse des Fermentationsprodukts hilfreich, sondern auch bei der Aufreinigung des Produkts von Nutzen.

### Beispiel 1

### Amplifizierung der Allele durch PCR

### A. Amplifizierung der cysE-Allele

Die im folgenden verwendeten cysE-Allele, cysEIV und cysEX, sind in DE 19539952 Beispiel 2/10 beschrieben.

Die Herstellung der dort erwähnten Mutationen ist durch die Verwendung der ortsspezifischen Mutagenese möglich. Kits zur Durchführung der Mutagenese sind im Handel beispielsweise von Stratagene (Stratagene GmbH, Postfach 105466, D-69044 Heidelberg) unter den Handelsnamen ExSite oder Chameleon erhältlich.

Nach Durchführung der ortsspezifischen Mutagenese wurden die erhaltenen Allele mittels der Polymerasekettenreaktion (PCR) (Saiki et al. 1988, Science 239: 487-491) aus der jeweiligen DNS mittels folgender Primer amplifiziert.

Die PCR-Experimente wurden in 30 Zyklen in Gegenwart von 200 µM Deoxynukleotidtriphosphaten (dATP, dCTP, dGTP, dTTP), je 1 µM des entsprechenden Oligonukleotids, 100 ng Matrizen-DNS mit dem jeweiligen cysE-Allel, 1/10 10fach Reaktionspuffer (100 mM KCl, 100 mM (NH₄)₂SO₄, 200 mM Tris-HCl (pH 8,8), 20 mM MgSO₄, 1 % Triton X-100 und 1000 µg/ml BSA) und 2,5 Einheiten einer hitzestabilen, rekombinanten Pfu-DNS-Polymerase (Stratagene) in einem Thermocycler (Gene-ATAQ-Controler, Pharmacia) unter folgenden Bedingungen durchgeführt: 94°C für 1 min, 60 °C für 1 min und 72 °C für 3 min.

Das Amplifizierungsprodukt wurde mit SacI und NsiI (beide von Boehringer Mannheim GmbH) unter den vom Hersteller angegebenen Bedingungen hydrolysiert, über ein 1 % Agarosegel aufgetrennt und mit Hilfe der Geneclean-Methode (Geneclean Kit BIO101 P.O. Box 2284 La Jolla, California, 92038-2284) nach den Angaben des Herstellers aus dem Agarosegel als etwa 1,0 kb großes Fragment isoliert. Bis zur weiteren Verwendung wurde das Fragment bei -20°C gelagert.

### B. Amplifizierung des mar-Locus

Der mar-Locus von Escherichia coli wurde mittels PCR amplifiziert. Das Verfahren zur Gewinnung der Amplifikate ist dasselbe, wie es in Beispiel 1 Abschnitt A beschrieben wurde. Als Matrizen-DNS wurde die chromosomale DNS aus Escherichia coli W3110 (ATCC 27325) verwendet. Als Matrizen DNS ist das Plasmid 100-1-1 (DSM 11545) ebenso geeignet. Die Lyse der Zellen und die Reinigung der chromosomalen DNS erfolgte nach dem in Ausubel et al., 1987, 2.4.1 - 2.4.2, Current Protocols in Molecular Biology, Greene Publishing Associates and Wiley-Interscience, beschriebenen Protokoll. Folgende Primer wurden für die Amplifizierung des mar-Locus verwendet:

Die Amplifizierung des mar-Locus führte zu einem etwa 3 kb großen Fragment, das wie in Beispiel 1 Abschnitt A beschrieben gereinigt wurde. Der nachfolgende Restriktionsverdau wird mit den Enzymen AscI und PacI (beide von New England Biolabs GmbH, Postfach 2750, D-65820 Schwalbach/Taunus) gemäß den Anleitungen und mit den Puffern des Herstellers durchgeführt. Nach der Reinigung des Fragments durch Agarosegelelektrophorese wurde es bei -20°C gelagert.

### C. Amplifizierung der ORF306-DNS

Die für den ORF306 kodierende DNS wurde wie in Beispiel 1 Abschnitt A beschrieben über PCR amplifiziert. Als Matrizen-DNS wurde die in Beispiel 1 Abschnitt B isolierte chromosomale DNS aus E. coli W3110 (ATCC 27325) verwendet. Als Matrizen-DNS ebenso geeignet ist das Plasmid 100-1-1 (DSM 11545). Die verwendeten Primer sind folgende:

Das amplifizierte DNS-Fragment ist etwa 1,05 kb groß und wurde, wie beschrieben, durch Agarosegelelektrophorese gereinigt. Ein anschließender Restriktionsverdau mit den Enzymen AsnI (Boehringer Mannheim) und PacI (New England Biolabs) führte nach einer Enzymentfernung zum gewünschten DNS-Fragment. Dies wurde bis zur Verwendung bei -20°C gelagert.

### D. Amplifizierung des für den GAPDH-Promotor kodierenden DNS Fragments

Zur wirksamen Transkription des ORF306 wurde der Promotor des Glycerinaldehyd-3-phosphatdehydrogenasegens verwendet. Dieses gewünschte DNS-Fragment wurde ebenfalls mittels PCR gewonnen.

Als Matrizen-DNS diente wieder die chromosomale DNS aus Escherichia coli W3110 (ATCC 27325). Als Matrizen-DNS ebenso geeignet ist das Plasmid 100-1-1. Es wurden folgende Primer verwendet:

Das erhaltene DNS Fragment mit etwa 0,3 kb wurde durch eine Agarosegelelektrophorese isoliert und gereinigt, wie in Bsp. 1 Abschnitt A beschrieben. Ein anschließender Restriktionsverdau mit den Enzymen MluI und PacI führte zum gewünschten DNS-Fragment. Nach der Entfernung der Restriktionsenzyme wurde die DNS bei -20°C gelagert.

### Beispiel 2

### Konstruktion der erfindungsgemäßen Plasmide

Als Basisplasmid zur Konstruktion der erfindungsgemäßen Plasmide wurde das Plasmid pACYC184 verwendet. Dieses Plasmid wurde modifiziert, wie in DE 19539952 beschrieben, und als Plasmid pACYC184-LH unter der Hinterlegungsnummer DSM 10172 bei der Deutschen Sammlung für Mikroorganismen in Braunschweig hinterlegt.

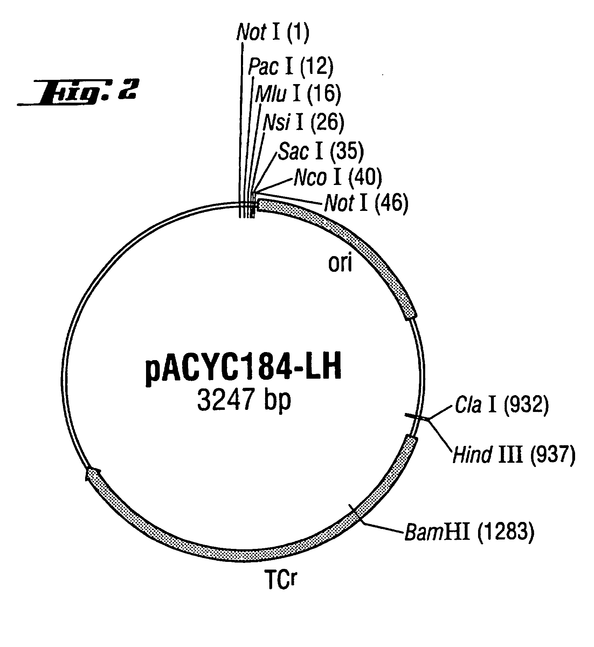

Eine Restriktions- und Funktionskarte von Plasmid pACYC184-LH ist in Figur 2 gezeigt. Das Plasmid pACYC184-LH trägt einen Polylinker. Dieser Polylinker weist folgende Restriktionsschnittstellen auf:

In diesen Linker wurden die in Beispiel 1 durch PCR und anschließenden Restriktionsverdau gewonnenen DNS-Fragmente ligiert.

### A. Konstruktion der Kontrollplasmide pACYC184/cysEIV und pACYC184/cysEX

Die Herstellung der Plasmide pACYC184/cysEIV und pACYC184/cysEX ist in der DE 19539952 Bsp 3 beschrieben und im folgenden kurz zusammengefaßt:

Etwa 1 µg des Plasmids pACYC184-LH (DSM 10172) wurde mit den Restriktionsenzymen SacI und NsiI nach den Angaben des Herstellers (Boehringer Mannheim) verdaut. Die verdaute DNS wurde anschließend durch eine Agarosegelelektrophorese zur Entfernung der Enzyme gereinigt, wie dies vorher beschrieben wurde. Die in Beispiel 1 Abschnitt A gewonnenen DNS-Fragmente, die für das jeweilige cysE-Allel kodieren, wurden dann mit dem SacI und NsiI verdauten Plasmid pACYC184-LH äquimolar gemischt und mit 1 µl T4 DNS Ligase und 2 µl 10fach Ligasepuffer (beides Boehringer Mannheim) versetzt und mit sterilem, doppelt destilliertem H₂O auf ein Gesamtvolumen von 20 µl aufgefüllt. Das Gemisch wurde bei 4°C über Nacht inkubiert und zur Transformation von Escherichia coli W3110 (ATCC 27325) verwendet. Das im folgenden beschriebene Transformationsverfahren wurde bei allen in den Beispielen genannten Transformationen angewendet.

Die Transformation von E. coli W3110 erfolgte mittels Elektroporation. Hierzu wurden 500 ml LB-Medium (10 g Trypton, 5 g Hefeextrakt, 5 g NaCl) in einem 1 l Erlenmeyerkolben mit 1 % (V/V) einer Übernachtkultur in demselben Medium angeimpft. Nach einer Inkubation im Rundschüttler bei 37°C bis zu einer optischen Dichte von 0,5-0,6 bei 600 nm wurden die Zellen durch eine Zentrifugation bei 4°C in einem sterilen Becher geerntet. Alle weiteren Schritte wurden nun auf Eis und unter Einhaltung steriler Bedingungen durchgeführt. Das Zellpellet wurde nun zweimal mit 500 ml eiskaltem, sterilem, doppelt destilliertem H₂O gewaschen und schließlich in 30 ml 10 % (V/V) sterilem Glycerin resuspendiert. Nach einer weiteren Zentrifugation wurde das Zellpellet in 500 µl 10 % (V/V) Glycerin aufgenommen und in 200 µl Aliquots bei -80°C gelagert. Für die Transformation wurden die Zellen auf Eis aufgetaut, mit etwa 10-100 ng DNS versetzt und in eine sterile Elektroporationsküvette (BioRad) gegeben. Die Küvette wurde in den Gene Pulser (BioRad) gestellt und bei einer Spannung von 2500 Volt, einem Parallelwiderstand von 200 Ohm und einer Kapazität von 25 µF elektroporiert. Anschließend wurden die Zellen in 1 ml SOC Medium (Caseinpepton 20,0 g/l, Hefeextrakt 5,0 g/l, NaCl 0,58 g/l, KCl 0,19 g/l, MgCl₂ 2,03 g/l, MgSO₄ 2,46 g/l, Glukose 3,60 g/l, pH = 7,0) resuspendiert und für eine Stunde bei 37°C geschüttelt. Danach wurden die Zellen entsprechend verdünnt, auf LB-Agarplatten (10 g/l Trypton, 5 g/l Hefeextrakt, 5 g/l NaCl, 15 g/l Agar, pH = 7,2) ausplattiert und über Nacht bei 37°C inkubiert, bis Einzelkolonien sichtbar wurden.

Die gewünschten Transformanden wurden nach einer Plasmidisolierung mittels QIAprep Spin Plasmid Kit (Qiagen GmbH, Max-Volmer-Straße 4, D-40724 Hilden) durch eine Restriktionsanalyse identifiziert. Sie wurden in Beispiel 3 als Kontrolle in der Fermentation verwendet.

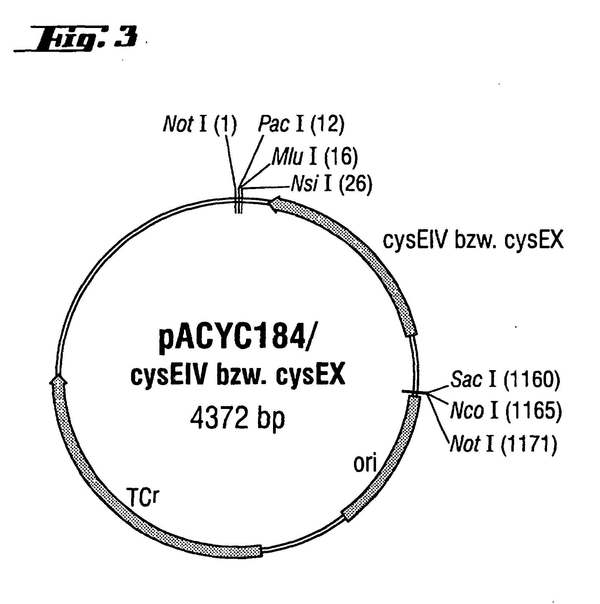

Die Plasmide pACYC184/cysEIV und pACYC184/cysEX sind in Figur 3 dargestellt.

### B. Konstruktion der Plasmide pACYC184/cysEIV-mar und pACYC184/cysEX-mar

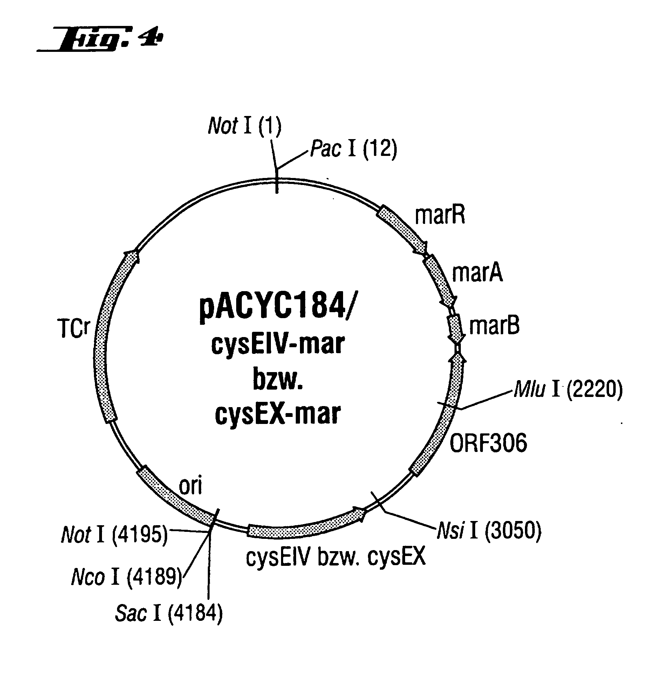

Jeweils 1 µg der in Beispiel 2 Abschnitt A konstruierten Plasmide pACYC184/cysEIV und pACYC184/cysEX wurde nacheinander mit den Restriktionsenzymen MluI (Boehringer) und PacI (New England Biolabs) gemäß den Angaben der Hersteller verdaut. Nach diesem Restriktionsverdau wurde die DNS durch eine Agarosegelelektrophorese isoliert und gereinigt, wie dies in Beispiel 1 Abschnitt A beschrieben wurde. Etwa 20 ng der MluI-PacI verdauten Vektoren pACYC184/cysEIV bzw. pACYC184/cysEX wurden jeweils mit 200 ng des in Beispiel 1 Abschnitt A hergestellten DNS Fragments, 1 µl T4 DNS Ligase (Boehringer Mannheim) und 2 µl 10fach Ligasepuffer (Boehringer Mannheim) und der erforderlichen Menge sterilem, doppelt destilliertem H₂O in einem Endvolumen von 20 µl gemischt. Nach einer Inkubation über Nacht bei 4°C wurden die zwei DNS-Gemische zur Transformation von Escherichia coli W3110 (ATCC 27325) verwendet. Nach einer Plasmidisolierung mittels QIAprep Spin Plasmid Kit (Qiagen GmbH) und einer Restriktionsanalyse wurden die gewünschten Transformanden isoliert und in der Fermentation eingesetzt, wie dies in Beispiel 3 beschrieben ist. Eine Restriktions- und Funktionskarte der Plasmide pACYC184/cysEIV-mar bzw. pACYC184/cysEX-mar ist in Figur 4 gezeigt.

### C. Konstruktion der Plasmide pACYC184/cysEIV-GAPDH und pACYC184/cysEX-GAPDH

Die in Beispiel 2 Abschnitt A konstruierten Plasmide pACYC184/cysEIV und pACYC184/cysEX wurden jeweils mit den Restriktionsenzymen MluI (Boehringer Mannheim) und PacI (New England Biolabs) gemäß den Angaben der Hersteller verdaut.

Nach der Reinigung dieser so behandelten Plasmide wurden jeweils mit dem in Beispiel 1 Abschnitt D hergestellten DNS Fragment zwei Ligationen angesetzt, wie dies in Beispiel 2 Abschnitt B beschrieben wurde.

Nach einer Inkubation über Nacht bei 4°C wurden die Ligationsansätze in E. coli W3110 transformiert. Die richtigen Transformanden wurden nach der Isolierung der Plasmid DNS durch eine Analyse mittels geeigneter Restriktionsenzyme identifiziert.

Die Plasmide pACYC184/cysEIV-GAPDH und pACYC184/cysEX-GAPDH wurden als Ausgangsmaterialien zur Konstruktion der Plasmide pACYC184/cysEIV-GAPDH-ORF306 und pACYC184/cysEX-GAPDH-ORF306 verwendet, wie dies im folgenden Abschnitt D beschrieben ist.

### D. Konstruktion der Plasmide pACYC184/cysEIV-GAPDH-ORF306 und pACYC184/cysEX-GAPDH-ORF306

Die in Abschnitt C hergestellten Plasmide wurden mit NdeI (Boehringer Mannheim) und PacI (New England Biolabs) gemäß den Angaben der Hersteller verdaut. Nach dem Reinigen der Plasmid DNS wurden mit dem AsnI-PacI geschnittenen DNS Fragment aus Beispiel 1 Abschnitt C, das für den ORF306 kodiert, zwei Ligationen angesetzt.

Nach einer Inkubation über Nacht bei 4°C wurde das DNS Gemisch jeweils in E. coli W3110 transformiert und auf LB-Platten ausplattiert. Nach dem Erscheinen der Einzelkolonien wurden diese durch Plasmidisolierung und Restriktionsverdau auf ihre Richtigkeit hin überprüft.

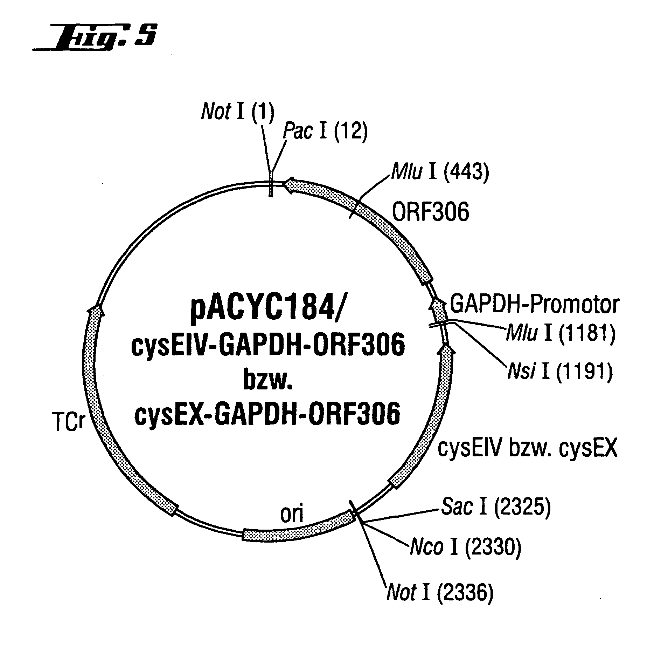

Eine Restriktions- und Funktionskarte der Plasmide pACYC184/cysEIV-GAPDH-ORF306 und pACYC184/cysEX-GAPDH-ORF306 ist in der Figur 5 gezeigt.

### Beispiel 3

### Vergleich der Ausbeuten der erfindungsgemäßen Konstrukte und bekannter Konstrukte in der Fermentation

Alle in der Fermentation verglichenen Plasmide wurden in E. coli W3110 fermentiert. So ist gewährleistet, daß die jeweils beobachteten Ausbeutesteigerungen nur aus der erfindungsgemäßen Verwendung der Gene resultieren.

20 ml LB-Medium mit 15 mg/l Tetracyclin wurden in einem Erlenmeyerkolben (100 ml) mit dem jeweiligen E. coli Konstrukt beimpft. Nach siebenstündiger Inkubation im Schüttelinkubator (150 Upm, 30°C) wurden die jeweiligen Vorkulturen in 100 ml SM1-Medium überführt (12 g/l K₂HPO₄, 3 g/l KH₂PO₄, 5 g/l (NH₄)₂SO₄, 0,3 g/l MgSO₄ x 7 H₂O, 0,015 g/l CaCl₂ x 2 H₂O, 0,002 g/l FeSO₄ x 7 H₂O, 1 g/l Na₃Citrat x 2 H₂O, 0,1 g/l NaCl, 1 ml/l Spurenelementlösung, bestehend aus 0,15 g/l Na₂MoO₄ x 2H₂O, 2,5 g/l H₃BO₃, 0,7 g/l CoCl₂ x 6 H₂O, 0, 25 g/l CuSO₄ x 5 H₂O, 1,6 g/l MnCl₂ x 4 H₂O, 0,3 g/l ZnSO₄ x 7 H₂O), das mit 5 g/l Glucose, 5 mg/l Vitamin B1 und 15 mg/l Tetracyclin supplementiert war. Die Kulturen wurden in Erlenmeyerkolben (1 l) bei 30 °C für 17 h mit 150 Upm geschüttelt. Nach dieser Inkubation betrug die optische Dichte bei 600 nm (OD₆₀₀) zwischen 3 und 5.

Die Fermentation wurde in Fermentern BIOSTAT M der Firma Braun-Melsungen durchgeführt. Es wurde ein Kulturgefäß mit 2 l Gesamtvolumen verwendet. Das Fermentationsmedium enthält 15 g/l Glucose, 10 g/l Trypton (Difco), 5 g/l Hefeextrakt (Difco), 5 g/l (NH₄)₂SO₄, 1,5 g/l KH₂PO₄, 0,5 g/l NaCl, 0,3 g/l MgSO₄ x 7 H₂O, 0,015 g/l CaCl₂ x 2 H₂O, 0,075 g/l FeSO₄ x 7 H₂O, 1 g/l Na₃Citrat x 2 H₂O und 1 ml Spurenelementlösung (siehe oben), 0,005 g/l Vitamin B1 und 15 mg/l Tetracyclin. Der pH-Wert im Fermenter wurde zu Beginn durch Zupumpen einer 25% NH₄OH-Lösung auf 7,0 eingestellt. Während der Fermentation wurde der pH-Wert durch automatische Korrektur mit 25 % NH₄OH auf einem Wert von 7,0 gehalten. Zum Animpfen wurden 100 ml Vorkultur in das Fermentergefäß gepumpt. Das Anfangsvolumen betrug etwa 1 l. Die Kulturen wurden zunächst mit 200 rpm gerührt und mit 1,5 vvm einer über einen Sterilfilter entkeimten Preßluft begast. Die Luftsauerstoffsättigung wurde während der Fermentation auf 50 % eingestellt. Die Kontrolle erfolgte automatisch über die Rührgeschwindigkeit. Die Fermentation wurde bei einer Temperatur von 30 °C durchgeführt. Nach 2 h Fermentationszeit erfolgte eine Zufütterung aus einer sterilen 30 % Na-Thiosulfat x 5 H₂O - Stammlösung mit einer Rate von 3 ml pro Stunde. Nach Erreichen einer OD₆₀₀ von 10 wurde eine sterile 56 % Glukose-Stammlösung mit einer Rate von etwa 8 - 14 ml pro Stunde zudosiert. Die Bestimmung des Glukosegehalts erfolgte enzymatisch mit Hilfe eines Glukoseanalysators der Firma YSI. Die Glukosekonzentration wurde während der Fermentation durch kontinuierliches Zufüttern zwischen 10 und 20 g/l eingestellt. Der Gesamtgehalt an Cystein des Mediums wurde aus dem zellfreien Überstand der Probe colorimetrisch nach Gaitonde, M. K. (1967), Biochem. J. 104, 627 - 633, bestimmt. Hierbei ist zu beachten, daß das während der Fermentation in Lösung verbleibende Cystein vor allem als Thiazolidinderivat vorliegt, aber durch den Test trotzdem erfaßt wurde. Falls zur Umsetzung des gebildeten Cysteins zum Thiazolidinderivat das erforderliche Keton oder Aldehyd (in diesem Fall Pyruvat) nicht mehr in ausreichenden Mengen zur Verfügung steht, wird freies L-Cystein gebildet, das im Test ebenfalls miterfaßt wird. Bei einer Bildung von freiem L-Cystein wird dies durch den eingebrachten Luftsauerstoff während der Fermentation langsam zum L-Cystin oxidiert. Cystin ist im wäßrigen Milieu bei pH 7,0 schwer löslich und fällt als weißes Pellet aus. Bei Ausbildung eines unlöslichen Cystinpellets wurde dies nach Abtrennung des Überstands einer gezogenen Probe nach Zentrifugation in halbkonzentrierter HCl aufgelöst und ebenfalls unter reduzierenden Bedingungen (DTT) im oben erwähnten Test vermessen.

Unter diesen Bedingungen wurden nach einer Fermentationsdauer von 24 Stunden bzw. 48 Stunden die, in den Tabellen 1 und 2 gezeigten Ausbeuten erreicht.

**Tabelle 1**

| Ausbeuten an Gesamtcystein mit dem cysEIV-Allel | | | |
|---|---|---|---|
| | Ausbeuten an Gesamtcystein (g/l) mit folgenden Plasmidkonstrukten | | |
| Fermentationszeit | pACYC184/ cysEIV | pACYC184/ cysEIV-mar | pACYC184/cysEIV -GAPDH-ORF306 |
| 24 Stunden | 1 | 3,8 | 3,8 |
| 48 Stunden | 1,6 | 5 | 3,2 + 6,3* |

| | | | |
|---|---|---|---|
| *als Pellet vorhandene Cystinmenge in Gramm pro Liter | | | |

**Tabelle 2**

| Ausbeuten an Gesamtcystein mit dem cysEX-Allel | | | |
|---|---|---|---|
| | Ausbeuten an Gesamtcystein (g/l) mit folgenden Plasmidkonstrukten | | |
| Fermentationszeit | pACYC184/cysE X | pACYC184/cysEX -mar | pACYC184/cysEX -GAPDH-ORF306 |
| 24 Stunden | 4,9 | 5,9 | 12,8 |
| 48 Stunden | 6,8 | 11,4 | 7,2 + 12,0* |

| | | | |
|---|---|---|---|
| *als Pellet vorhandene Cystinmenge in Gramm pro Liter | | | |

### Beispiel 4

### Nachweis für die Bildung von 2-Methyl-thiazolidin-2,4-dicarbonsäure

Für den Nachweis der Bildung der 2-Methyl-thiazolidin-2,4-dicarbonsäure als Hauptprodukt der in Beispiel 3 beschriebenen Fermentation wurde das Konstrukt E. coli W3110 x pACYC184/cysEX-GAPDH-ORF306 wie in Beispiel 3 beschrieben fermentiert.

Nach 24 Stunden wurde der Fermentationsüberstand durch eine Zentrifugation von den Zellen abgetrennt. Die beschriebene Cysteinmessung ergab 12,8 g Gesamtcystein im Überstand. Der Fermentationsüberstand wurde dann mit MgSO₄ in einer Endkonzentration von 0,3 M versetzt. Nach einer Inkubation dieses Überstands über Nacht bei 4°C unter Rühren bildete sich ein weißer Niederschlag. Bei diesem Niederschlag handelte es sich um das schwerlösliche Magnesiumsalz der 2-Methyl-thiazolidin-2,4-dicarbonsäure.

Nach einer Abtrennung dieses Niederschlags durch Zentrifugation wurde im Überstand lediglich eine Restmenge an Cystein von 2,5 g/l gemessen.

Der Niederschlag wurde in halbkonzentrierter HCl aufgelöst und ebenfalls einem Cysteintest unterworfen. Hier fand sich eine Konzentration von 9,5 g/l Cystein. Nach einer ¹H und ¹³C NMR Untersuchung des in D₂O + HCl aufgelösten Niederschlags wurde dieser gegen eine Referenzsubstanz (M.P. Schubert, J. Biol. Chem. 121, 539-548 (1937)) als 2-Methyl-thiazolidin-2,4-dicarbonsäure indentifiziert.

### Beispiel 5

### Unterschiedliche Toxizitäten von L-Cystein und 2-Methyl-Thiazolidin-2,4(R)-dicarbonsäure

### Für diesen Versuch wurde

2-Methyl-Thiazolidin-2,4(R)-dicarbonsäure nach der Methode von Schubert (M.P. Schubert, J. Biol. Chem. 121, 539-548 (1937)) aus L-Cystein und Pyruvat synthetisiert. Eine Übernachtkultur von E. coli W3110 in LB-Medium wurde in 20 ml SM1-Medium (siehe Beispiel 3) geimpft, das mit 10 g/l Glucose, 10 % LB-Medium, 5 mg/l Vitamin B1, 15 mg/l Tetracyclin und jeweils entsprechenden Mengen L-Cystein oder 2-Methyl-Thiazolidin-2,4(R)-dicarbonsäure supplementiert war. Nach einer 7 stündigen Inkubation bei 37 °C zeigte sich im mit L-Cystein versetzten Medium kein Wachstum mehr ab 1 mM, während im, mit 2-Methyl-Thiazolidin-2,4(R)-dicarbonsäure versetzten, Medium ein Wachstum bis zu 50 mM zu beobachten war. Längere Inkubationszeiten waren aufgrund der leichten Oxidierbarkeit von Cystein nicht möglich. Somit ergibt sich bei
L-Cystein eine deutlich höhere Toxizität für E. coli als bei 2-Methyl-Thiazolidin-2,4(R)-dicarbonsäure. 2-Methyl-Thiazolidin-2,4(R)-dicarbonsäure eignet sich daher deutlich besser zur Gewinnung von L-Cystein durch fermentative Verfahren, auch wenn hierbei noch ein chemischer Schritt zur Freisetzung des L-Cysteins notwendig ist.

### Beispiel 6

### Verstärkte Bildung von N-Acetyl-L-Serin

Die Bildung von N-Acetyl-L-Serin erfolgt durch spontane Umlagerung aus O-Acetyl-L-Serin. Dieses O-Acetyl-L-Serin ist die unmittelbare Vorstufe von L-Cystein im Biosyntheseweg bei Bakterien. Bei unzureichendem oder fehlendem Schwefeleinbau in O-Acetyl-L-Serin ist das Endprodukt einer solchen Fermentation somit N-Acetyl-L-Serin. Die erfindungsgemäßen Gene erhöhen bei fehlender Schwefelzufuhr auch die Ausbeute an diesem Fermentationsprodukt.

Die in Beispiel 3 beschriebene Fermentation wurde ohne Thiosulfatfütterung durchgeführt. Die verwendeten Konstrukte, die die Wirksamkeit der vorliegenden Gene, insbesondere des ORF306 zeigen sollten, waren pACYC184/cysEX und pACYC184/cysEX-GAPDH-ORF306.

Wie die Ergebnisse in Tabelle 3 zeigen, erhöhen die erfindungsgemäßen Gene, insbesondere der ORF306, deutlich die Ausbeute an N-Acetyl-L-Serin in der Fermentation.

**Tabelle 3**

| Ausbeuten an N-Acetyl-L-Serin nach 24 h Fermentation | |
|---|---|
| Konstrukt | N-Acetyl-L-Serin (g/l) |
| pACYC184/cysEX | 7,6 |
| pACYC184/cysEX-GAPDH-ORF306 | 15,9 |

Bei Verwendung von stärker feedback-resistenten cysE-Allelen (beispielsweise cysEXIV, cysEXI und cysEXXII aus DE 19539952) in Kombination mit den erfindungsgemäßen Genen können Ausbeuten an N-Acetyl-L-Serin von über 30 g/l erreicht werden.

## Patentansprüche

1. Mikroorganismenstamm, der zur fermentativen Herstellung von L-Cystein, L-Cystin, N-Acetyl-Serin und/oder Thiazolidinderivaten geeignet ist, **dadurch gekennzeichnet, daß** er in seinem Cysteinstoffwechsel so dereguliert ist, daß er eine erhöhte Menge an L-Cystein bildet und mindestens ein Gen kodierend für ein Protein mit SEQ.ID.NO: 2 oder eine Variante davon, welche zur Herstellung von L-Cystein, L-Cystin, N-Acetyl-Serin und/oder Thiazolidinderivaten beiträgt, überexprimiert.

2. Mikroorganismenstamm gemäß Anspruch 1, **dadurch gekennzeichnet, daß** der Cysteinstoffwechsel durch ein feedbackresistentes Cys-E Allel dereguliert ist.

3. Protein umfassend die Sequenz oder eine Variante der Sequenz welche zur Herstellung von L-Cystein, L-Cystin, N-Acetyl-Serin und/oder Thiazolidinderivaten beiträgt.

4. Verfahren zur Herstellung von L-Cystein, L-Cystin, N-Acetyl-Serin oder Thiazolidinderivaten hiervon **dadurch gekennzeichnet, daß** ein Mikroorganismenstamm gemäß Anspruch 1 oder 2 in an sich bekannter Art und Weise in der Fermentation eingesetzt wird.

5. Verwendung von Proteinen gemäß Anspruch 3 zur verstärkten Expression von L-Cystein, L-Cystin, N-Acetyl-Serin, und oder Thiazolidinderivaten in der Fermentation.

## Claims

1. Microorganism strain which is suitable for the fermentative preparation of L-cysteine, L-cystine, N-acetylserine and/or thiazolidine derivatives, **characterized in that** its cysteine metabolism is deregulated such that it forms an increased quantity of L-cysteine and overexpresses at least one gene encoding a protein having the sequence SEQ. ID. No: 2 or a variant thereof which contributes to the preparation of L-cysteine, L-cystine, N-acetylserine and/or thiazolidine derivatives.

2. Microorganism strain according to Claim 1, **characterized in that** the cysteine metabolism is deregulated by a feedback-resistant Cys-E allele.

3. Protein which comprises the sequence or a variant of the sequence which contributes to the preparation of L-cystein, L-cystine, N-acetylserine and/or thiazolidine derivatives.

4. Process for preparing L-cysteine, L-cystine, N-acetylserine or thiazolidine derivatives thereof, **characterized in that** a microorganism strain according to Claim 1 or 2 is employed in the fermentation in a manner known per se.

5. Use of proteins according to Claim 2 for the augmented expression of L-cysteine, L-cystine, N-acetylserine and/or thiazolidine derivatives in the fermentation.

## Revendications

1. Souche de microorganismes, appropriée pour la production par fermentation de L-cystéine, L-cystine, N-acétyl-sérine et/ou de dérivés de thiazolidine, **caractérisée en ce qu'**elle est déréglée dans son métabolisme de cystéine de telle manière qu'elle forme une quantité plus élevée de L-cystéine et surexprime au moins un gène codant pour une protéine présentant la SEQ ID. NO:2 ou une variante de celle-ci, qui contribue à la production de L-cystéine, L-cystine, N-acétyl-sérine et/ou de dérivés de thiazolidine.

2. Souche de microorganismes selon la revendication 1, **caractérisée en ce que** le métabolisme de la cystéine est déréglé par un allèle cys-E résistant à la rétroaction.

3. Protéine comprenant la séquence ou une variante de la séquence qui contribue à la production de L-cystéine, L-cystine, N-acétyl-sérine et/ou de dérivés de thiazolidine.

4. Procédé pour la production de L-cystéine, L-cystine, N-acétyl-sérine ou de dérivés de thiazolidine de ceux-ci, **caractérisé en ce qu'**on utilise une souche de microorganismes selon la revendication 1 ou 2 de manière connue en soi dans la fermentation.

5. Utilisation de protéines selon la revendication 3 pour l'expression renforcée de L-cystéine, L-cystine, N-acétyl-sérine et/ou de dérivés de thiazolidine dans la fermentation.
